# DEMANDE DE BREVET EUROPEEN

(11) **EP 1 739 076 A1**
(43) Date de publication de la demande: **03.01.2007**
(21) Numéro de dépôt: 06116066.9
(22) Date de dépôt: 26.06.2006
(51) Int. Cl.: C07C 229/42, C07C 237/10, A61K 8/44

(54) **Nouvelles para-phénylènediamines doubles reliées par un bras de liaison substitué par un ou plusieurs radicaux carboxyliques et/ou dérivés et utilisation en coloration**

(30) Priorité: 29.06.2005 FR 0551810
(71) Demandeur: L'ORÉAL, 75008 Paris (FR)
(72) Inventeur: Bordier, Thierry, 93290 Tremblay en France (FR); Sabelle, Stéphane, 75005 Paris (FR)
(74) Mandataire: Fevrier, Murielle Françoise E.

(57) **Abrégé**

La présente demande concerne une nouvelle famille de para-phénylènediamines doubles reliées par un bras de liaison substitué par un ou plusieurs radicaux carboxyliques et/ou dérivés et leur utilisation pour la coloration des fibres kératiniques.

Ces nouvelles para-phénylènediamines sont utiles en tant que base d'oxydation pour la teinture des fibres kératiniques.

## Description

La présente demande concerne une nouvelle famille de para-phénylènediamines doubles reliées par un bras de liaison substitué par un ou plusieurs radicaux carboxyliques et/ou dérivés et leur utilisation pour la coloration des fibres kératiniques.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diaminobenzènes aromatiques, les méta-aminophénols, les méta-diphénols et certains composés hétérocycliques tels que des composés indoliques et pyridiniques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

Le but de la présente invention est de fournir de nouvelles bases d'oxydation capables de colorer les fibres kératiniques avec des nuances variées, puissantes, esthétiques et peu sélectives ainsi que des colorations résistantes aux diverses agressions que peuvent subir les fibres telles que la lumière, la sueur et les shampoings.

Ce but est atteint avec la présente invention qui a pour objet une nouvelle famille de para-phénylènediamine double de formule (I) suivante ainsi que les sels d'addition correspondants: 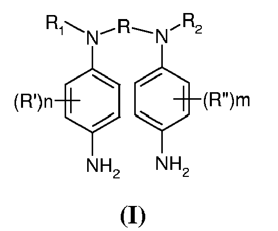 dans laquelle :
- R représente un radical alkylène en C₁-C₁₀ linéaire ou ramifié substitué par un ou plusieurs radicaux :
   - carboxyle
   - aminocarbonyle
   - (monoalkyl(C₁-C₆) ou dialkyl(C₁-C₆))aminocarbonyle
   - alkoxy(C₁-C₁₆)carbonyle
- R₁ et R₂ représentent, indépendamment l'un de l'autre,
   - un atome d'hydrogène
   - un radical alkyle en C₁-C₆ linéaire ou ramifié.
   - un radical alkyle en C₁-C₆ linéaire ou ramifié substitué par un ou plusieurs radicaux hydroxy, alkoxy en C₁-C₄, amino, monoalkyl amino en C₁-C₄, dialkylamino en C₁-C₄
- R' et R" représentent, indépendamment l'un de l'autre,
   - un radical alkyle en C₁-C₆,
   - un radical alcoxy en C₁-C₆,
   - un radical hydroxy-alcoxy en C₁-C₆,
   - un radical alcoxy(C₁-C₆)alkyle en C₁-C₆,
   - un radical mono ou poly-hydroxy alkyle en C₁-C₆
- n et m représentent, indépendamment l'un de l'autre, un entier variant de 0 à 4 à l'exception de

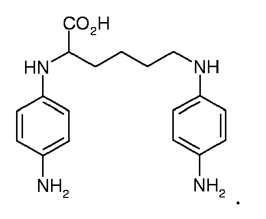

La composition de la présente invention permet en particulier d'obtenir une coloration de fibres kératiniques très puissante, peu sélective et tenace, en particulier à la lumière.

Un autre objet de l'invention concerne des compositions pour la coloration des fibres kératiniques, en particulier des fibres kératiniques humaines telles que les cheveux, comprenant au moins une para-phénylènediamine de formule (I).

L'invention a aussi pour objet un procédé de coloration mettant en oeuvre cette composition, l'utilisation de la composition selon la présente invention pour la coloration des fibres kératiniques, en particulier les fibres kératiniques humaines telles que les cheveux et un dispositif à plusieurs compartiments ou "kit" de teinture.

A titre d'exemple, on peut citer les para-phénylènediamines suivantes :

| | |
|---|---|
| 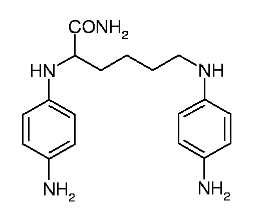 | 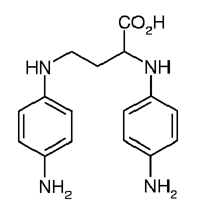 |
| 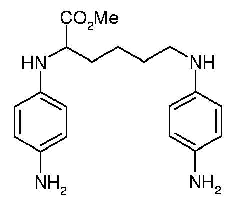 | 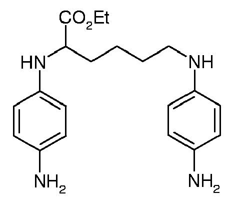 |
| 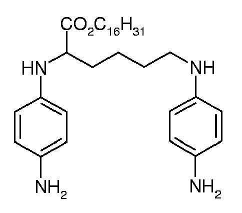 | 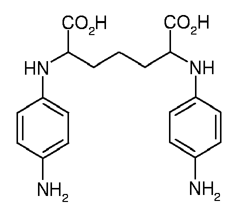 |

Selon un mode de réalisation particulier, les para-phénylènediamines de formule (I) sont telles que R₁ et R₂ représentent indépendamment un atome d'hydrogène ou un groupe alkyle en C₁-C₄ pouvant être substitué. R représente de préférence Un radical alkylène en C₂-C₇ linéaire ou ramifié substitué par un ou plusieurs radicaux alkoxy(C₁-C₄)carbonyle, carboxyle, amino-carbonyle. Selon un mode de réalisation particulier, n et m sont égal à 0 ou 1.

D'une manière générale, les sels d'addition utilisables sont notamment choisis parmi les sels d'addition avec un acide, tels que l'acide chlorhydrique, l'acide bromhydrique, l'acide sulfurique, l'acide citrique, l'acide succinique, l'acide tartrique, l'acide lactique, l'acide para-toluènesulfonique, l'acide benzènesulfonique, l'acide phosphorique et l'acide acétique.

Ils peuvent aussi être sous forme de solvates par exemple un hydrate ou un solvate d'alcool linéaire ou ramifié tel que l'éthanol ou l'isopropanol.

Les para-phénylènediamines de formule (I) selon la présente demande peuvent être préparés suivant une méthode de synthèse classique. On pourra par exemple se reporter à la demande de brevet DE10144226A.

A titre d'illustration, les para-phénylènediamines de formule (I) peuvent être synthétisés selon le schéma réactionnel suivant :

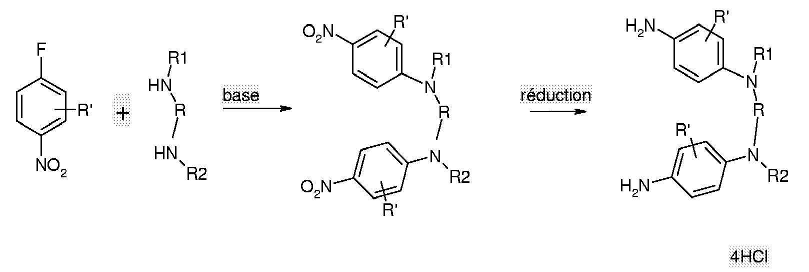

La première étape de la synthèse est une substitution nucléophile d'une diamine sur un dérivé de para-fluoro-nitrobenzène, étape inspirée des publications Synthesis 1990 (12), 1147-1148 et Synth. Commun. 1990, 20 (22), 3537-3545. La deuxième étape est une étape de réduction classique, pouvant être par exemple une réaction d'hydrogénation par catalyse hétérogène en présence de Pd/C, Pd(II)/C, Ni de Raney ou encore une réaction de réduction par un métal, par exemple par du zinc, fer, étain... (Advanced Organic Chemistry, 4th edition, 1992, J. MARCH, WILEY Interscience ; Rreduction in Organic Chemistry, M. Hudlicky, 1983, Ellis Honwood series Chemical Science).

La présente demande concerne aussi les composés nitrés de formule (II) suivante qui peuvent être utilisés pour l'obtention des para-phénylènediamines de formule (I): 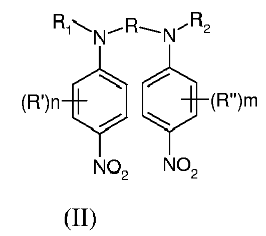 dans laquelle R₁, R₂, R', R", R, n et m sont tels que définis précédemment.

L'invention a aussi pour objet une composition de coloration comprenant dans un milieu approprié à la coloration au moins une base d'oxydation du type para-phénylènediamine de formule (I) telle que définie précédemment.

La quantité en para-phénylènediamine de formule (I) dans la composition de teinture est en général comprise entre 0,0001% et 20% en poids par rapport au poids total de la composition, de préférence entre 0,01% et 10%.

La composition selon l'invention contient de préférence au moins un coupleur d'oxydation.

Parmi les coupleurs d'oxydation, on peut notamment citer les méta-phénylènediamines, les méta-aminophénols, les méta-diphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'a-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement, la quantité du ou des coupleurs d'oxydation est comprise entre 0,0001 et 20%, de préférence entre 0,005 à 6% en poids par rapport au poids total de la composition.

La composition selon l'invention peut également contenir au moins une base d'oxydation additionnelle différente des bases d'oxydation de formule (I).

Les bases d'oxydation peuvent notamment être choisies parmi les para-phénylènediamines, les bis-phénylalkylènediamines, les para--aminophénols, les ortho-aminophénols et les bases hétérocycliques et leurs sels d'addition.

Parmi les para-phénylènediamines, on peut plus particulièrement citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, le 2 thiényl paraphénylène diamine, le 2-β hydroxyéthylamino 5-amino toluène, la 3-hydroxy 1-(4' aminophenyl)pyrrolidine, la 6-(4-Amino-phenylamino)-hexan-1-ol et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, la 6-(4-Amino-phenylamino)-hexan-1-ol et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4'-aminophényl) tétraméthylènediamine, la N,N'-bis-(β-hydroxyéthyl) N,N'-bis-(4'-aminophényl) tétraméthylènediamine, la N,N'-bis-(4'-amino-3'-méthylphényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le paraaminophénol, le 4-amino 3-méthyl phénol, le 4-amino 3-fluoro phénol, le 4-amino 2-chloro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino-2,6-dichlorophénol, le 4-amino-6[((5'-amino-2'-hydroxy-3'-méthyl)phényl)méthyl]-2-méthylphénol, le bis(5'-amino-2'-hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, le 5-[(2-hydroxyéthyl)amino]-2-méthylphenol et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1026978 et GB 1153196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2733749 et DE 19543988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration de la ou des bases d'oxydation additionnelle est comprise entre 0,0001 et 20%, de préférence entre 0,005 à 6% en poids par rapport au poids total de la composition.

La composition tinctoriale conforme à l'invention peut en outre contenir un ou plusieurs colorants directs pouvant notamment être choisi parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, la composition selon l'invention comprend au moins un colorant choisi parmi les colorants directs cationiques et les colorants directs naturels.

Parmi les colorants directs cationiques utilisables selon l'invention, on peut citer les colorants directs azoïques cationiques décrits dans les demandes de brevets WO 95/15144, WO-95/01772 et EP-714954.

Parmi ces composés, on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

Le milieu approprié pour la coloration est avantageusement constitué par de l'eau ou par un mélange d'eau et d'au moins un solvant organique tel que, par exemple, les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

Les solvants sont, de préférence, présents dans des proportions de préférence comprises entre 1 et 40% en poids environ par rapport au poids total de la composition tinctoriale, et encore plus préférentiellement entre 5 et 30% en poids environ.

Avantageusement, la composition de coloration comprend au moins un adjuvant cosmétique choisi parmi les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

Les adjuvants ci-dessus sont en général présents en quantité comprise pour chacun d'eux entre 0,01 et 20 % en poids par rapport au poids de la composition.

Le pH de la composition conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques autres que les diacides carboxyliques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule : 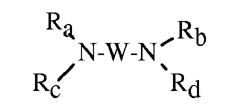 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

Bien entendu, l'homme de l'art veillera à choisir le ou les adjuvants, précurseurs de colorants d'oxydations additionnels, coupleurs d'oxydation, colorants directs de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de la présente demande concerne un procédé de coloration des fibres kératiniques dans lequel on applique sur les fibres la composition de l'invention telle que définie précédemment, et on révèle la couleur à l'aide d'un agent oxydant. La couleur peut être révélée à pH acide, neutre ou alcalin. L'agent oxydant peut être ajouté à la composition de l'invention juste au moment de l'emploi. Il peut être mis en oeuvre à partir d'une composition oxydante le contenant, appliquée simultanément ou séquentiellement à la composition de l'invention.

A titre d'agents oxydants, on peut citer le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases, le peroxyde d'hydrogène étant particulièrement préféré.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

La composition oxydante peut renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, notamment humaines et en particulier les cheveux humains.

La présente demande concerne également l'utilisation de la composition cosmétique selon l'invention comprenant, dans un milieu approprié pour la teinture, au moins une para-phénylènediamine de formule (I) pour la teinture des fibres kératiniques, de préférence les fibres kératiniques humaines telles que les cheveux.

L'invention a aussi pour objet un dispositif à plusieurs compartiments ou "kit" de coloration dans lequel un premier compartiment renferme une composition tinctoriale contenant une para-phénylènediamine de formule (I) et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

### EXEMPLES DE SYNTHESE

### EXEMPLE 1 : Synthèse du tétrachlorhydrate de l'éthyl 2,6-bis[(4-aminophényl)amino]hexanoate (3)

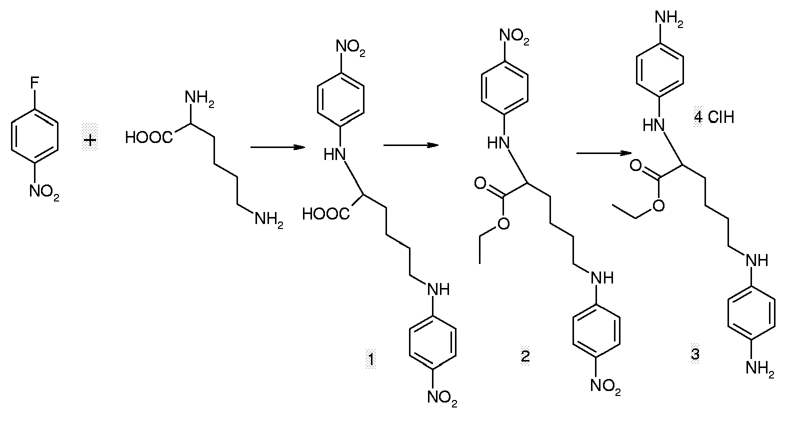

### Etape 1 : synthèse de l'acide 2,6-bis[(4-nitrophényl)amino]hexanoique (1)

Dans un tricol de 250 ml muni d'un réfrigérant et d'un thermomètre, 5 g (34,2 mmoles) de L-lysine sont dissous dans 100 ml d'eau en présence de 1,4 g (1éq.) de soude et 8,6 g (3éq.) de bicarbonate de soude. Une solution de 10,8 ml (3éq.) de 4-fluoro-nitrobenzène dans 60 ml d'éthanol est versée au mélange lequel est porté au reflux (85°-90°C) pendant 5 jours. Le mélange refroidi est extrait avec de l'éther éthylique. La phase aqueuse est acidifiée jusqu'à pH~3 par de l'acide chlorhydrique 5N. Un précipité gommeux se forme lequel est extrait par du dichlorométhane en présence d'un peu de méthanol. La phase organique est lavée à l'eau jusqu'à neutralité, séchée sur sulfate de sodium puis évaporée pour donner une huile orangée qui cristallise en présence d'éther éthylique. On obtient 11,7 g d'un produit cristallin jaune soit un rendement de 88%.

### Etape 2 : synthèse de l'éthyl 2,6-bis[(4-nitrophényl)amino]hexanoate (2)

Dans un tricol de 100 ml muni d'un réfrigérant et d'un thermomètre, on introduit 5 g d'acide 2,6-bis[(4-nitrophenyl)amino]hexanoique dissous dans 50 ml d'éthanol et 1,5 ml d'acide sulfurique, puis on chauffe au reflux pendant une nuit.

Le mélange homogène est ensuite refroidi puis évaporé à sec. Le résidu est repris par du dichlorométhane puis la phase organique résultante est lavée par une solution saturée aqueuse 1N d'hydrogénocarbonate de sodium, puis par de l'eau.

Après séchage sur du sulfate de sodium, la phase organique est évaporée à sec. On obtient un solide jaune d'une masse de 2,9 g avec un rendement de 54%.

### Etape 3 : synthèse du tétrachlorhydrate de l'éthyl 2,6-bis[(4-aminophenyl) amino]hexanoate (5)

Dans un hydrogénateur de 250 ml, on introduit 2 g d'éthyle 2,6-bis[(4-nitrophenyl)amino]hexanoate dissous dans 100 ml d'un mélange d'isopropanol/eau (7/3) en présence de 4 g de palladium sur charbon.

Après introduction d'hydrogène pendant une heure à 53°C et sous 10 bars, le milieu réactionnel est filtré puis concentré sous vide.

Ce milieu est repris par un mélange d'isopropanol /acétone/HCl (4N) puis on filtre le précipité obtenu et on le lave à l'éther éthylique. On obtient 1,8 g de tétrachlorhydrate d'éthyl 2,6-bis[(4-aminophenyl) amino]hexanoate (5) avec un rendement de 75%.

Les spectres RMN du proton et de masse sont conformes à la structure attendue du produit.

### EXEMPLE 2 : Synthèse du tétrachlorhydrate de l'acide 2,6-bis[(4-aminophényl)amino] heptanedioique (5)

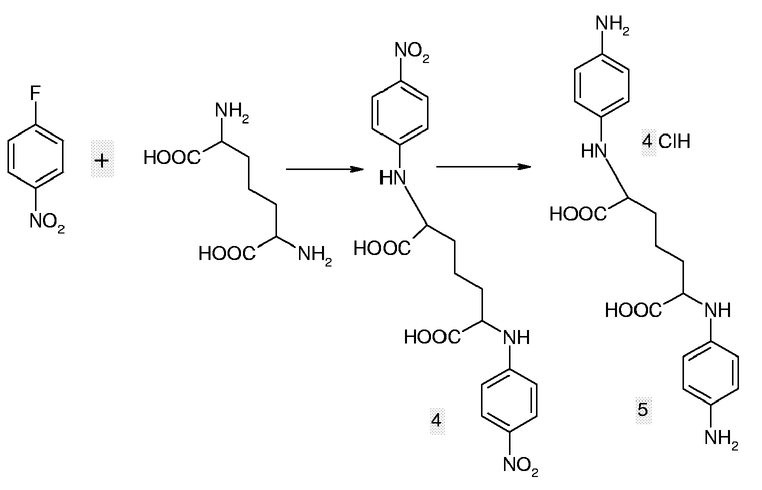

### Etape 1 : synthèse de l'acide 2,6-bis[(4-nitrophényl)amino]heptanedioique (4)

Dans un tricol de 250 ml, on introduit 5 g d'acide 2,6 diaminopinélique dissout dans 50 ml d'eau, 2,1 g de soude (2éq.) et 6,6 g d'hydrogénocarbonate de sodium. Puis 8,4 ml de 4-fluoro nitrobenzène en solution dans 50 ml d'éthanol sont versés dans le mélange et on porte la réaction au reflux pendant 4 jours. Le mélange est ensuite refroidi, la phase aqueuse est acidifiée à pH=3 par HCl 1N. Un dépôt huileux marron se forme ; le milieu réactionnel est extrait avec du dichlorométhane et de l'éthanol. On ajoute ensuite de l'éther éthylique pour amorcer la cristallisation. Après filtration et séchage du solide formé, on obtient 7,7 g d'un solide orange avec un rendement de 68%.

### Etape 2: synthèse dutétrachlorhydrate de l'acide 2,6-bis[(4-aminophényl)amino] heptanedioique (5)

Dans l'hydrogénateur de 250 ml, on introduit 3 g d'acide 2,6-bis[(4-nitrophenyl)amino]heptanedioique et 3 g de palladium sur charbon dans un mélange 70/30 ml éthanol/eau. Le produit est hydrogéné sous 7 bars pendant 1 heure à température ambiante. Sous azote, le mélange est soutiré et récupéré dans 50 ml d'eau et 5,5 ml HCl 5N ; on filtre sur célite puis lave à l'eau.

Le filtrat est évaporé à sec puis repris dans l'acétone. Le solide est alors filtré et lavé à l'acétone, puis à l'éther éthylique.

Après séchage, on obtient 2,4 g d'un solide crème avec un rendement de 67%.

Les spectres RMN du proton et de masse sont conformes à la structure attendue du produit.

### EXEMPLE 3 : Synthèse du tétrachlorhydrate de 2,6-bis[(4-aminophenyl)amino]hexanamide (7)

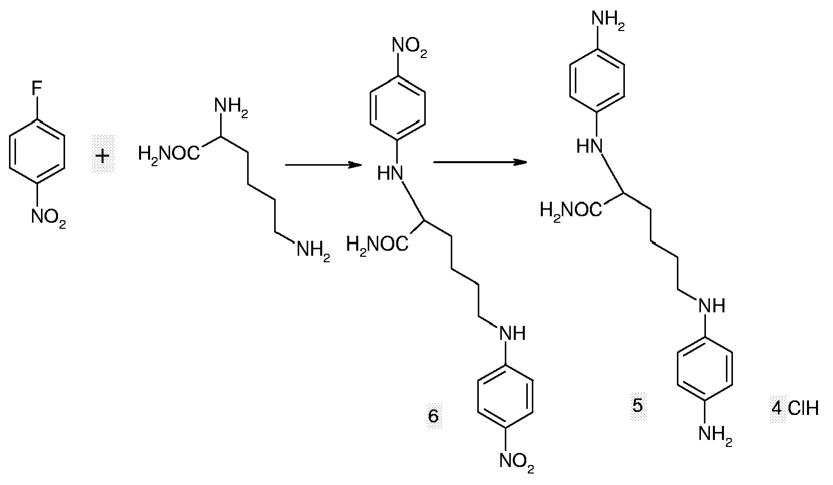

### Etape 1 : synthèse de l'acide 2,6-bis[(4-nitrophényl)amino]heptanedioique (4)

2,3 g de 4-fluoronitrobenzène (2 éq.) sont dissous dans 10 ml de DMSO. 1,2 équivalent de 2,6-diaminohexanamide et 4 équivalents de triéthylamine sont ajoutés à la solution. Le milieu réactionnel est porté à 60°C pendant 24 heures. Le mélange est ensuite versé sur de la glace pilée, un précipité se forme. Ce dernier est filtré, lavé à l'eau, puis séché.

### Etape 2: synthèse du tétrachlorhydrate du 2,6-bis[(4-aminophenyl)aminol hexanamide (5)

Dans un hydrogénateur de 250 ml, on introduit 0,815 mg d'acide 2,6-bis[(4-nitrophényl)amino]heptanedioique (4) et 140 mg de palladium sur charbon dans 100 ml éthanol. Le produit est hydrogéné sous 7 bars pendant 1 heure à température ambiante. Sous azote, le mélange est soutiré et récupéré dans 50 ml d'isoppropanol et 5,5 ml d'isopropanol chlorhydrique. Après ajout d'éther isopropylique dans le filtrat, le solide est filtré sur célite puis lavé à l'acétone et à l'éther éthylique.

Après séchage, on obtient 755 mg d'un solide crème.

Les spectres RMN du proton et de masse sont conformes à la structure attendue du produit.

### Exemples de teinture

### Exemples 1 à 14 : Composition tinctoriale à partir du tétrachlorhydrate du 2,6-bis[(4-aminophenyl)amino]hexanamide (7)

### Exemples 1 à 7 : Teinture en milieu acide

Les compositions tinctoriales suivantes sont préparées :

| **Exemple** | **1** | **2** | **3** | **4** | **5** | **6** | **7** |
|---|---|---|---|---|---|---|---|
| tétrachlorhydrate du 2,6-bis[(4-aminophenyl)amino]hexanamide (7) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mole | | | |
| 3,6-Dimethyl-1H-pyrazolo[5,1-c][1,2 ,4]triazole | | | | | 10-3 mole | | |
| chlorhydrate du 2-(2,4-Diamino-phenoxy)-ethanol, | | | | | | 10-3 mole | |
| chlorhydrate du 3-Amino-2-chloro-6-methyl-phenol, | | | | | | | 10-3 mole |
| Support de teinture (2) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **1** | **2** | **3** | **4** | **5** | **6** | **7** |
|---|---|---|---|---|---|---|---|
| **Nuance observée** | gris intense | gris violet intense | gris violet-rouge intense | brun rouge intense | gris violet-rouge intense | bleu intense | violet intense |

### Exemples 8 à 14 : teinture en milieu basique

Les compositions tinctoriales suivantes sont préparées :

| **Exemple** | **8** | **9** | **10** | **11** | **12** | **13** | **14** |
|---|---|---|---|---|---|---|---|
| tétrachlorhydrate du 2,6-bis[(4-aminophenyl) amino]hexanamide (7) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mole | | | |
| 3,6-Dimethyl-1H-pyrazolo[5,1-c][1,2 ,4]triazole | | | | | 10-3 mole | | |
| chlorhydrate du 2-(2,4-Diamino-phenoxy)-ethanol, | | | | | | 10-3 mole | |
| chlorhydrate du 3-Amino-2-chloro-6-methyl-phenol, | | | | | | | 10-3 mole |
| Support de teinture (1) | (*) | (*) | (*) | (*) | (*) | (*) | (*) |
| Eau déminéralisée q.s.p. | 100g | 100g | 1 00g | 100g | 100g | 1 00g | 100g |

| | | | | | | | |
|---|---|---|---|---|---|---|---|
| (*) : support de teinture (2) p**H** 9.5 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| **Exemple** | **8** | **9** | **10** | **11** | **12** | **13** | **14** |
|---|---|---|---|---|---|---|---|
| **Nuance observée** | brun orangé | violet-rouge intense | rouge | brun rouge | rouge chromatique intense | bleu intense | violet intense |

## Revendications

1. Para-phénylènediamine de formule (I) suivante et sels d'addition correspondants : 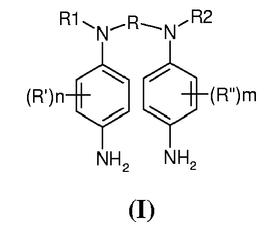 dans laquelle :
• R représente un radical alkylène en C₁-C₁₀ linéaire ou ramifié substitué par un ou plusieurs radicaux :
- carboxyle
- aminocarbonyle
- (monoalkyl(C₁-C₆) ou dialkyl(C₁-C₆))aminocarbonyle
- alkoxy(C₁-C₁₆)carbonyle
• R₁ et R₂ représentent, indépendamment l'un de l'autre,
- un atome d'hydrogène
- un radical alkyle en C₁-C₆ linéaire ou ramifié.
- un radical alkyle en C₁-C₆ linéaire ou ramifié substitué par un ou plusieurs radicaux hydroxy, alkoxy en C₁-C₄, amino, monoalkyl amino en C₁-C₄, dialkylamino en C₁-C₄
• R' et R" représentent, indépendamment l'un de l'autre,
- un radical alkyle en C₁-C₆,
- un radical alcoxy en C₁-C₆,
- un radical hydroxy-alcoxy en C₁-C₆,
- un radical alcoxy(C₁-C₆)alkyle en C₁-C₆,
- un radical mono ou poly-hydroxy alkyle en C₁-C₆
• n et m représentent, indépendamment l'un de l'autre, un entier variant de 0 à 4 à l'exception de

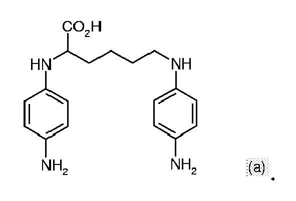

2. Para-phénylènediamine selon la revendication 1 dans laquelle R représente un radical alkylène en C₂-C₇ linéaire ou ramifié substitué par un ou plusieurs radicaux alkoxy(C₁-C₄)carbonyle, carboxyle, aminocarbonyle.

3. Para-phénylènediamine selon la revendication 1 ou 2 dans laquelle R₁ et R₂ représentent indépendamment un atome d'hydrogène ou un groupe alkyle en C₁-C₄ pouvant être substitué.

4. Para-phénylènediamine selon l'une quelconque des revendications 1 à 3 dans laquelle n et m représentent indépendamment 0 ou 1.

5. Para-phénylènediamine selon l'une quelconque des revendications 1 à 4 dans laquelle les sels d'addition avec un acide des para-phénylènediamines de formule générale (I) sont choisis parmi l'acide chlorhydrique, l'acide bromhydrique, l'acide sulfurique, l'acide citrique, l'acide succinique, l'acide tartrique, l'acide lactique, l'acide para-toluènesulfonique, l'acide benzènesulfonique, l'acide phosphorique et l'acide acétique, ces composés pouvant éventuellement être sous forme de solvates.

6. Para-phénylènediamine selon l'une quelconque des revendications 1 à 5 choisie parmi :
| | |
|---|---|
| 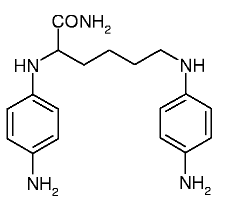 | 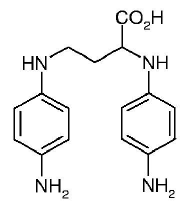 |
| 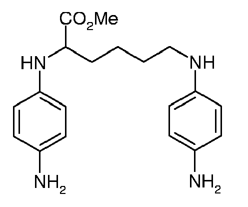 | 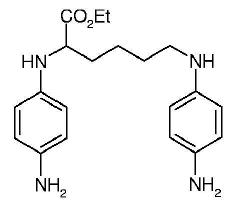 |
| 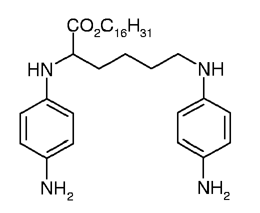 | 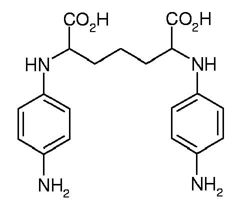 |

7. Para-phénylènediamine selon la revendication 6 choisies parmi :
| | |
|---|---|
| 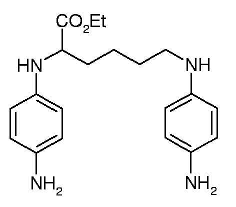 | 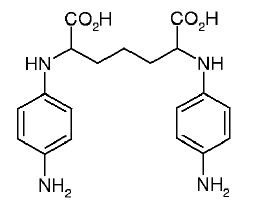 |
| 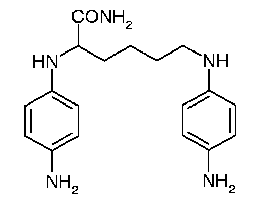 | |

8. Composition de coloration comprenant, dans un milieu approprié à la coloration, au moins une base d'oxydation para-phénylènediamine de formule (I) telle que définie précédemment aux revendications 1 à 7 à l'exception de celle de formule (a).

9. Composition selon la revendication 8 dans laquelle la quantité en para-phénylènediamine de formule (I) est comprise entre 0,0001% et 20% en poids par rapport au poids total de la composition, de préférence entre 0,01% et 10%.

10. Composition selon la revendication 8 ou 9 comprenant de plus au moins un coupleur d'oxydation.

11. Composition selon la revendication 10 dans laquelle le coupleur d'oxydation est choisi parmi les méta-phénylènediamines, les méta-aminophénols, les méta-diphénols, les coupleurs naphtaléniques et les coupleurs hétérocycliques ainsi que leurs sels d'addition.

12. Composition selon l'une quelconque des revendications 8 à 11 comprenant de plus au moins une base d'oxydation additionnelle différente des bases d'oxydation de formule (I).

13. Composition selon la revendication 12 dans laquelle les bases d'oxydation peuvent être choisies parmi les para-phénylènediamines, les bis-phénylalkylènediamines, les para-aminophénols, les ortho-aminophénols et les bases hétérocycliques et leurs sels d'addition.

14. Composition selon l'une quelconque des revendications 8 à 13 comprenant au moins un colorant direct.

15. Composition selon l'une quelconque des revendications 8 à 14 dans laquelle le milieu approprié pour la teinture est constitué par de l'eau contenant éventuellement au moins un solvant organique.

16. Composition selon la revendication 15 dans laquelle le solvant organique est choisi parmi les alcools inférieurs en C₁-C₄, ramifiés ou non, ainsi que les alcools aromatiques et leurs mélanges.

17. Composition selon l'une quelconque des revendications 8 à 16 comprenant au moins un adjuvant cosmétique choisi parmi les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

18. Composition selon la revendication 17 dans laquelle la quantité en adjuvant cosmétique est comprise pour chacun d'eux entre 0,01 et 20 % en poids par rapport au poids total de la composition.

19. Composition selon l'une quelconque des revendications précédentes comprenant un agent oxydant.

20. Procédé de coloration des fibres kératiniques **caractérisée par le fait qu'**on applique sur les fibres au moins une composition selon l'une quelconque des revendications 8 à 18 en présence d'un agent oxydant pendant un temps suffisant pour développer la coloration désirée.

21. Dispositif à plusieurs compartiments dans lequel un premier compartiment contient une composition tinctoriale pour la coloration des fibres kératiniques telle que définie à l'une quelconque des revendications 8 à 18 et un deuxième compartiment contient un agent oxydant.

22. Procédé de préparation des para-phénylènediamines de formule (I) à l'exception de celle de la formule (I) par réduction des composés nitrés de formule (II) : 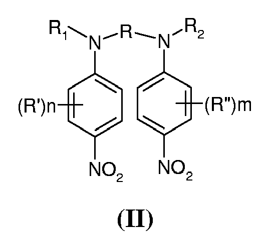 dans laquelle R₁, R₂, R, R', R", n, m sont tels que définis aux revendications 1 à 7.

23. Composé nitré de formule (II) 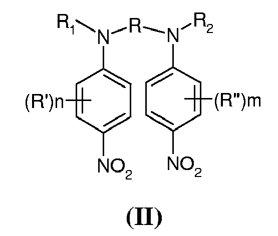 dans laquelle R₁, R₂, R, R', R", n, m sont tels que définis aux revendications 1 à 7 à l'exception de
